# EUROPEAN PATENT APPLICATION

(11) **EP 1 605 385 A1**
(43) Date of publication of application: **14.12.2005**
(21) Application number: 04013603.8
(22) Date of filing: 09.06.2004
(51) Int. Cl.: G06F 19/00

(54) **Method and apparatus for an automated and self-controlled quantification system of molecular biological images and functions**

(71) Applicant: Thymed GmbH, 55116 Mainz (DE); Kayser, Klaus, Prof. Dr., 69121 Heidelberg (DE)
(72) Inventor: Kayser, Klaus, 69121 Heidelberg (DE); Kayser, Gian, 79108 Freiburg (DE); Radziszowski, Dominik, 30-658 Krakow (PL)
(74) Representative: Krauss, Jan

(57) **Abstract**

The present invention relates to a method of self controlling and automated quantification (measures) of images and functions which have been created by molecular-biological, live imaging or other medical methods. The system permits the quantification of individual (patient-related) and/or serial (experiment-related) image (function) information. The output format can be designed by the user. The body of the system includes an internet portal, platform-independent program tools which control and operate on data (image) control, data (image) standardisation, and transfer, as well as specialised (data) image and function measuring program tools, and connected image data banks. The measurement programs provide the clients with stereological, object-oriented, and structure-related data.

## Description

The present invention relates to a method of self controlled and automated quantification (measuring) of images and functions which have been created by molecular-biological, live imaging or other medical methods. The inventive system permits the quantification of individual (patient-related) and/or serial (experiment-related) image (function) information. The output format can be designed by the user. The body of the inventive system includes an internet portal, platform-independent program tools which control and operate on data (image) control, data (image) standardisation, and transfer, as well as specialised (data) image and function measuring program tools, and connected image data banks. The measurement programs provide the clients with stereological, object-oriented, and structure-related data. The present invention therefore generally relates to a self-controlling and automated quantification of image information content in diagnostic medicine, especially in molecular biology and immunohistochemistry.

For the purposes of the present invention, all references as cited herein are herewith incorporated by reference in their entireties.

Progress in medicine is related to the refinement of understanding of molecular biological and molecular genetic processes which are closely associated with occurrence and development of the disease under investigation. A prerequisite for these analyses is a non-biased sampling and measuring of data, which are obtained during occurrence and development of the disease.

Basically, life is characterised by interactions of functions and structures, and, consecutively, non-biased data of health and disease are presented in images and functions. Thus, all modem diagnostic techniques such as radiology, pathology, endoscopy, etc. are involved in viewing and analysing images and functions. Live imaging can include computerised tomography, chest X-rays, nuclear resonance techniques, and isotope tomography (PET). Immunohistochemistry and in situ hybridisation are imaging techniques most frequently used in diagnostic pathology, other approaches furthermore need interpretation of low magnified images (endoscopy, dermatology, etc.).

Viewing an image and/or the extraction of image information requires the "detection" of objects, i.e., a segmentation of the image into object and the background. There are several measuring systems commercially available, which can be used for these purposes; however, they are all embedded in a non-standardised environment and connected with specialised measuring systems, i.e., bound to the standards used by the respective company. In addition, they do require specialised knowledge and training of a potential user.

Kayser et al. (Kayser K, Beyer M, Blum S, Kayser G. Anal Cell Pathol. 2000;21(3-4):101-6. Recent developments and present status of telepathology.) describe telepathology which is the diagnostic work of a pathologist at a distance and can be classified in relation to application, technical solutions, or performance conditions. Applications comprise, amongst others, expert consultations or remote control measurements. The technical solutions distinguish active (remote control, live imaging) systems from passive (conventional microscope handling, static imaging), and the performance systems with interactive (on-line, live imaging) use from those with passive (offline, static imaging) practice. Intra-operative frozen section service is mainly performed with remote control systems; whereas expert consultations and education/training are commonly based upon Internet connections with static imaging in an off-line mode. Image size, line transfer rate and screen resolution define the practicability of the system. Several telepathology systems are described as being implemented in large Institutes of Pathology which serve for frozen section diagnosis in small hospitals located in the local area. Similarly, Kayser K (in J Telemed Telecare. 2002;8(6):325-30. Interdisciplinary telecommunication and expert teleconsultation in diagnostic pathology: present status and future prospects.) furthermore describes telepathology as being performed in an active or a passive manner.

Brauchli et al (in Brauchli K, Helfrich M, Christen H, Jundt G, Haroske G, Mihatsch M, Oberli H, Oberholzer M. The future of telepathology. An Internet "distributed system" with "open standards" Pathologe. 2002 May;23(3):198-206.) describe the telepathology system iPath that is based on small, but when possible independently working modules. Four software modules are available for a free and as fast as possible application: (1) the module "Microscope control", (2) the module "Connector" (insertion of images directly from the microscope without a motorized microscope), (3) the module "Client-application" via the web-browser and (4) the module "Server" with a database. The server is placed in the internet and not behind a firewall. The server permanently receives information from the periphery and returns the information to the periphery on request. The only thing which the expert, the non-expert and the microscope have to know is how contact can made with the server.

Blackmore et al. (Blackmore CC, Richardson ML, Linnau KF, Schwed AM, Lomoschitz FM, Escobedo EM, Hunter JC, Jurkovich GJ, Cummings P. Web-based image review and data acquisition for multiinstitutional research. AJR Am J Roentgenol. 2003 May; 180(5): 1243-6.) describe a user-friendly Web-based interface that allows review of images combined with integrated data collection and entry for use at multiple sites involved in a large multicenter research project.

Fernandez-Bayo et al. (Fernandez-Bayo J, Barbero O, Rubies C, Sentis M, Donoso L. Distributing medical images with internet technologies: a DICOM web server and a DICOM java viewer. Radiographics. 2000 Mar-Apr;20(2):581-90.) describe filmless radiology approaches based on World Wide Web technologies involving a Web server that allows the query and retrieval of images stored in a Digital Imaging and Communications in Medicine (DICOM) archive. The system is described as working well, especially with magnetic resonance and computed tomographic images in a filmless hospital environment.

Fieguth and Simpson (Fieguth P, Simpson T. Automated measurement of bulbar redness. Invest Ophthalmol Vis Sci. 2002 Feb;43(2):340-7.) describe a World Wide Web-based survey for the measurement of bulbar redness. They come to the conclusion that clinical grading of redness images is highly variable.

WO 01/11548 describes a method and system that are provided to report the findings of an expert's analysis of image data. The method and system are based on a reporting system that forms the basis of an image management system that can efficiently and systematically generate reports, facilitate data entry into searchable databases for data mining, and expedite billing and collections for the expert's services. The expert identifies a significant finding on an image and attaches a location-description code to the location of that finding in order to create a significant finding and an entry into a database. Further descriptions of that finding, such as dimensional measurements, audio descriptions, 3D rendered snapshots, etc., may be automatically appended to the finding as secondary attributes of the finding within the database. At the end of the expert's evaluation of the image(s), the system sorts the findings in the database and presents the findings by prioritized categories. The expert edits and approves a multimedia report, which may be delivered to an Internet server for immediate access, archived in the database, sent by automated voice, fax or e-mail to an end-user, or any combination thereof.

US 5,428,690 describes a method and apparatus for an automated assay of biological specimens positioned on microscope slides.

Although the above mentioned methods and systems show that already a significant progress has been achieved in telepathology, several needs in this area are still not fulfilled. The present invention addresses these needs. It is therefore the object of the present invention, to provide diagnostic medical specialists such as pathologists, radiologists, endoscopists, and researchers with an improved method for analysing images as well as an improved system for analysis, wherein the system a) operates on an open platform, b) can be used without any specific knowledge, c) offers the most frequently used measurements, d) is able to create standardised results, which can be transferred to hospital information systems (e.g. DICOM 3), e) creates a user-specific image databank, f) possesses self-controlling modules, and g) works in a completely automated manner.

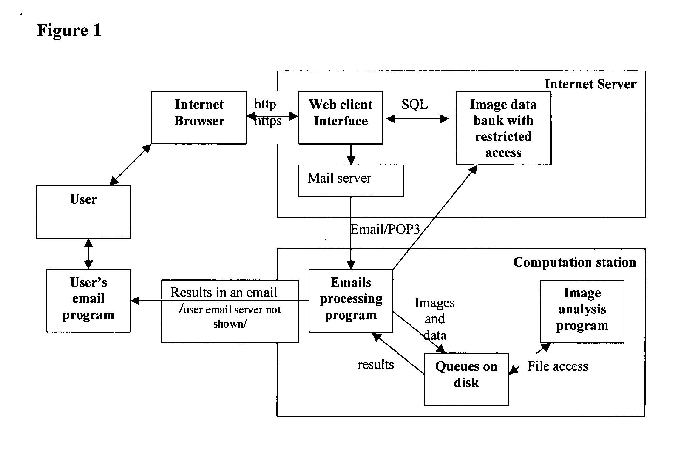

According to a first aspect of the present invention, the above problem is solved by a method for the automated analysis of biological images, said method comprising the steps of a) collecting and providing biological image data to first output means, b) transmitting said image data from said first output means to input processing means, c) processing said image data by said input processing means, d) transmitting said processed image data to automated image analysis means, e) further processing said processed image data by said automated image analysis means to generate image analysis result data, f) transmitting said image analysis result data to output processing means, and g) further transmitting said image analysis result data to second output means. In a preferred embodiment of the present invention, the body of the system for performing the inventive method consists of an internet browser web interface, an email processing program, an image analysing program, and an image databank (see Figure 1).

Preferred is a method according to the present invention that is characterised in that the biological image data is derived from immunohistochemical images, in situ hybridisation images, immunofluorescence images, radiological images or endoscopical images. Nevertheless, images that are derived from other biologically derived measurements can be used as long as they can be suitably converted into a format that can be analysed using the present method.

The formats of the images to be analysed are preferably either .bmp or .jpg. All other suitable image formats can be used as long as they can be suitably converted into a format that can be read and analysed using the present method.

Even more preferred is a method according to the present invention, wherein the first output means comprises an interface for accessing and sending data. In one particular embodiment, the interface for accessing and sending data comprises an internet browser. The internet browser can constitute one part of an internet platform structure that is provided by the present method. Even more preferred is a method according to the present invention, wherein that said transmitting of said image data to said input processing means comprises filling out a form with data necessary for measurement and report.

The internet platform can provide the client with an internet browser interface for sending and accessing data. It can include a form to be filled in with data necessary for measurement and report. The interface can check filled data correctness and accuracy as well as images' format (bmp and jpg). The data can be sent as an email and collected in an internal system email queue for further processing. The proposed solution does not required the computational station to be on-line all the time. It also improves scalability and load balancing of the system as many computational stations may be used for data processing.

According to a preferred method of the present invention, the image data is collected and further processed in an internal system email queue of said first output means. Said first output means can be constantly on-line or not constantly on-line.

In yet another preferred method according to the present invention, said input processing means performs steps selected from the group consisting of receiving messages with data, extracting data, converting image data into an appropriate format for further measurements, checking filled data correctness and accuracy as well as images format, transferring the data to the measurement input paths, administrating the image analysis means and serving for security tasks in the communication.

Most preferred is a method according to the present invention, wherein said input processing means comprises an email processing program. The email processing program can receive email messages with data, extracts data and converts images to an appropriate format for measurements (.bmp), transfer the files to the chosen measurement input paths (disk queue), and controls the output paths for re-submitting the results to the client. In addition, it can administrate the image data bank and serve for security tasks in the internet communication (user authorisation).

The present method and system can be applied via the internet by a specific server address. Then, the method and system preferably requires an identification of the user (pathologist, researcher, clinician) by his name, institution, and a personal security key. Then, only registered individuals/institutions have access to the system. After successful registration, the client can act as follows: a) setup of preferences and/or b) submit data for measurement. An authorised user can be, for example, identified based on his/her email address and personal security key. User administration can be performed manually but is preferably based on the web form as above. New users will have to fill the form and after his/her personal data verification a personal security code will be given and the user will have an access to the system.

In addition, the input processing means (e.g. e-mail processing program) can act for control of correct email messages, check the correct information content and provide the data security. It transfers the input data (images to be measured) into a system-internal standard and makes the images and data available for access by the measurement program.

Optionally and preferably, the input processing means (e.g. e-mail processing program) can act both as an input and output gate of the input data and measurement results. The measurement results are then re-transferred to the e-mail processing program, which then feeds the data into an image databank, provides the client-oriented form of the results, and submits the results to the client via email.

In yet another aspect of the method according to the present invention, the automated image analysis means performs steps comprising configuring the necessary file data, starting a loop checking the input disk and path for incoming data and image files, leaving the loop for file checking, performing the requested measurement, and creating an output file with the image analysis result data.

"Automated" in the context of the present invention means that the present method is performed substantially without human intervention, once the data to be analysed is fed to the first output means. Of course, even this step could preferably be automated, thus creating a "fully automated" method and system of the present invention. Automated, nevertheless, does include some manual interaction with the system, e.g. for maintenance purposes, for example for upgrading technical (system) and/or software components.

In another aspect of the method according to the present invention, said method is characterised in that configuring the necessary file data comprises checking the disks and paths of input and output files, the format of the files to be searched for, and the configuration of the data set up.

In another aspect of the method according to the present invention, the output processing means comprises the steps of picking up the file from said automated image analysis means, and translating said file into a suitable standard. It is preferred, that the suitable standard is DICOM.

In one preferred embodiment, the measurement program starts with the configuration of its necessary file data. These include the disks and paths of input and output files, the format of the files to be searched for, and the configuration of the data set up (clients data and translation of, for example, DICOM format). Having configured these internal standards, a loop is started which checks the input disk and path for incoming data and image files. The data file provides the program with the necessary measurement information, i.e. ID number, separation symbol for antibodies (#) and image numbers (_), measurement procedure, and image calibration. The program then leaves the loop for file checking, performs the wanted measurement, and creates an output file with the measurement data. This file will be picked up by the platform program, translated into DICOM standard, and sent to the client via internet (email attachment). Images, input, output and control data are then included into an image data bank.

During the whole measurement procedure a control file can be created which is erased after finishing the measurement. Having performed the measurements, a pertinent control message is implemented at the control path, which serves for documentation and which is included into the internal image data bank together with the measurement request, result and image files.

The internal loop is started again after determination of the measurement. The checking period of the loop is automatically adjusted to the time needed for measurement, i.e., if the measurement time lasts long, the checking time is prolonged. An input file checking time of 10 minutes is standard.

In another aspect of the method according to the present invention, the output processing means (e.g. the e-mail processing program) will re-transfer the measurement results into an image databank which provides the client-oriented form of the results, and submits the results to the client via email.

In another preferred embodiment of the method according to the present invention, said transmitting said image analysis result data to the client takes place via the internet and/or as an email attachment. Another preferred embodiment of the method according to the present invention is characterised in that said second output means comprises an image data bank, an interface or a monitor.

In one preferred embodiment, the program then leaves the loop for file checking, performs the wanted measurement, and creates an output file with the measurement data. This file will be picked up by the platform program, translated into DICOM standard, and sent to the client via internet (email attachment). Images, input, output and control data are then included into an image data bank.

A preferred method according to the present invention is characterised in that the interface comprises an internet browser. Yet another preferred method according to the present invention is characterised in that said method is performed substantially without human intervention. "Substantially without human intervention" in the context of the present invention means that the present method in its main components is performed without human intervention, once the program is started. Substantially, nevertheless, does include some human interaction with the system if required, e.g. for maintenance purposes, for example for upgrading technical (system) and/or software components.

In yet another aspect of the method according to the present invention, said method further comprises the steps of a) an automated internal and/or external control of image measuring systems by internally controlling of features of segmented areas according to the aim of the measurement, wherein objects with features are detected that do not match the features of the expected objects (or set of objects), and b) providing the client with a respective output information. Preferably, the inventive method further comprises controlling of internal "program failure" by an appropriate program reaction, for example, a stop of the measurement, if an internal error occurs and a restart of the program. More preferably, the restart of the program comprises either a new measurement or "internal repair".

Another general aspect of the present invention relates to a data processing system for automatically analysing biological images, said system comprising a) means for collecting and providing biological image data to first output means, b) means for transmitting said image data from said first output means to input processing means, c) means for processing said image data by said input processing means, d) means for transmitting said processed image data to automated image analysis means, e) means for further processing said processed image data by said automated image analysis means to generate image analysis result data, f) means for transmitting said image analysis result data to output processing means, and g) means for further transmitting said image analysis result data to second output means. In a preferred embodiment of the present invention, the body of the system for performing the inventive method consists of an internet browser web interface, an email processing program, an image analysing program, and an image databank (see Figure 1).

Preferred is a system according to the present invention that is characterised in that the biological image data is derived from immunohistochemical images, in situ hybridisation images, immunofluorescence images, radiological images or endoscopical images. Nevertheless, images that are derived from other biologically derived measurements can be used as long as they can be suitably converted into a format that can be analysed using the present system.

The formats of the images to be analysed are preferably either .bmp or .jpg. All other suitable image formats can be used as long as they can be suitably converted into a format that can be read and analysed using the present system.

Even more preferred is a system according to the present invention, wherein the first output means comprises an interface for accessing and sending data. In one particular embodiment of the system according to the present invention the interface for accessing and sending data comprises an internet browser. The internet browser can constitute one part of an internet platform structure that is provided by the present system. Even more preferred is a system according to the present invention, wherein transmitting of said image data to said input processing means comprises filling out a form with data necessary for measurement and report.

The internet platform can provide the client with an internet browser interface for sending and accessing data. It can include a form to be filled in with data necessary for measurement and report. The interface can check filled data correctness and accuracy as well as images format (bmp and jpg). The data can be sent as an email and collected in an internal system email queue for further processing. The proposed solution does not required the computational station to be on-line all the time. It also improves scalability and load balancing of the system as many computational stations may be used for data processing.

According to a preferred system according to the present invention, the image data is collected and further processed in an internal system email queue of said first output means. Said first output means can be constantly on-line or not constantly on-line.

In yet another preferred system according to the present invention said input processing means performs steps selected from the group consisting of receiving messages with data, extracting data, converting image data into an appropriate format for further measurements, checking filled data correctness and accuracy as well as images format, transferring the data to the measurement input paths, administrating the image analysis means and serving for security tasks in the communication.

Most preferred is a system according to the present invention, wherein said input processing means comprises an email processing program. The email processing program can receive email messages with data, extracts data and converts images to an appropriate format for measurements (.bmp), transfer the files to the chosen measurement input paths (disk queue), and controls the output paths for re-submitting the results to the client. In addition, it can administrate the image databank and serve for security tasks in the internet communication (user authorisation).

The present system can be embodied in the internet at a specific server address. Then, the system preferably requires an identification of the user (pathologist, researcher, clinician) by his name, institution, and a personal security key. Then, only registered individuals/institutions have access to the system. After successful registration, the client can act as follows: a) setup of preferences and/or b) submit data for measurement. An authorised user can be, for example, identified based on his/her email address and personal security key. User administration can be performed manually but is preferably based on the web form as above. New user will have to fill the form and after his/her personal data verification personal security code will be given and the user will have an access to the system.

In addition, the input processing means (e.g. e-mail processing program) can act for control of correct email messages, check the correct information content and provide the data security. It transfers the input data (images to be measured) into a system-internal standard and makes the images and data available for access by the measurement program.

Optionally and preferably, the input processing means (e.g. e-mail processing program) can act both as an input and output gate of the input data and measurement results. The measurement results are then re-transferred to the e-mail processing program, which then feeds the data into an image databank, provides the client-oriented form of the results, and submits the results to the client via email.
In yet another aspect of the system according to the present invention, said automated image analysis means performs steps comprising configuring the necessary file data, starting a loop checking the input disk and path for incoming data and image files, leaving the loop for file checking, performing the requested measurement, and creating an output file with the image analysis result data.

In another aspect of the system according to the present invention, said configuring the necessary file data comprises means for checking the disks and paths of input and output files, the format of the files to be searched for, and the configuration of the data set up.

In another aspect of the method according to the present invention, the output processing means comprises the steps of picking up the file from said automated image analysis means, and translating said file into a suitable standard. It is preferred, that the suitable standard is DICOM.

In one preferred embodiment, the measurement program starts with the configuration of its necessary file data. These include the disks and paths of input and output files, the format of the files to be searched for, and the configuration of the data set up (clients data and translation of, for eample, DICOM format). Having configured these internal standards, a loop is started which checks the input disk and path for incoming data and image files. The data file provides the program with the necessary measurement information, i.e. ID number, separation symbol for antibodies (#) and image numbers U, measurement procedure, and image calibration. The program then leaves the loop for file checking, performs the wanted measurement, and creates an output file with the measurement data. This file will be picked up by the platform program, translated into DICOM standard, and sent to the client via internet (email attachment). Images, input, output and control data are then included into an image data bank.

During the whole measurement procedure a control file can be created which is erased after finishing the measurement. Having performed the measurements, a pertinent control message is implemented at the control path, which serves for documentation and which is included into the internal image data bank together with the measurement request, result and image files.

The internal loop is started again after determination of the measurement. The checking period of the loop is automatically adjusted to the time needed for measurement, i.e., if the measurement time lasts long, the checking time is prolonged. An input file checking time of 10 minutes is standard.

In another aspect of the method according to the present invention, the output processing means (e.g. the e-mail processing program) will re-transfer the measurement results into an image databank which provides the client-oriented form of the results, and submits the results to the client via email.

In another aspect of the invention, the system further comprises means for transmitting said image analysis result data to the client via the internet and/or as an email attachment. Another preferred embodiment of the method according to the present invention is characterised in that said second output means comprises an image data bank, an interface or a monitor.

In one preferred embodiment, the program then leaves the loop for file checking, performs the wanted measurement, and creates an output file with the measurement data. This file will be picked up by the platform program, translated into DICOM standard, and sent to the client via internet (email attachment). Images, input, output and control data are then included into an image data bank.

A preferred system according to the present invention is characterised in that the interface comprises an internet browser. Yet another preferred system according to the present invention is characterised in that said system is functioning substantially without human intervention. "Substantially without human intervention" in the context of the present invention means that the present method in its main components is performed without human intervention, once the program is started. Substantially, nevertheless, does include some human interaction with the system if required, e.g. for maintenance purposes, for example for upgrading technical (system) and/or software components.

In yet another aspect of the system according to the present invention, said method further comprises a) means for an automated internal and/or external control of image measuring systems by internally controlling of features of segmented areas according to the aim of the measurement, wherein objects with features are detected that do not match the features of the expected objects (or set of objects), and b) means for providing the client with a respective output information. Preferably, the inventive system further comprises means for controlling of internal "program failure" by an appropriate program reaction, for example, a stop of the measurement, if an internal error occurs and a restart of the program. More preferably, the restart of the program comprises means for either a new measurement or "internal repair".

Another general aspect of the present invention relates to a computer program product comprising program code means stored on a tangible medium readable by a computer, the code means to execute the method according to the description above, when executed in the computer.

Another general aspect of the present invention relates to a computer readable storage medium that comprises a program according to the description above in the form of a computer readable program code. Suitable storage media are known to the person of skill and include, for example, CD-ROMs, diskettes, and ZIP-disks.

The invention has been fully implemented for analysis of immunohistochemical and in situ hybridization images (including immunofluorescence). It and can be easily extended for additional purposes such as radiological images, endoscopical images, etc.

The following examples relate to specific preferable embodiments of the present invention. The examples are provided for illustrative purposes only and shall not limit the scope of the present invention.

### In the accompanying Figures:

Figure 1 schematically shows the body of the system as described in Example 1. In the present example, the body of the system consists of an internet browser web interface, an email processing program, an image analysing program, and an image databank.

Figure 2 schematically shows the body of the measurement program structure and system ("computation station") as depicted in Figure 1.

Figure 3 represents a screenshot of an example of an internet browser interface according to the present invention.

Figure 4 represents a screenshot of an example of the presented screen that is shown if the data are in preparation for measurement.

### Examples

### Basic program structure

The body of the system is schematically depicted in Figure 1. The body of the system consists of an internet browser web interface, an email processing program, an image analysing program, and an image databank.

### Internet platform

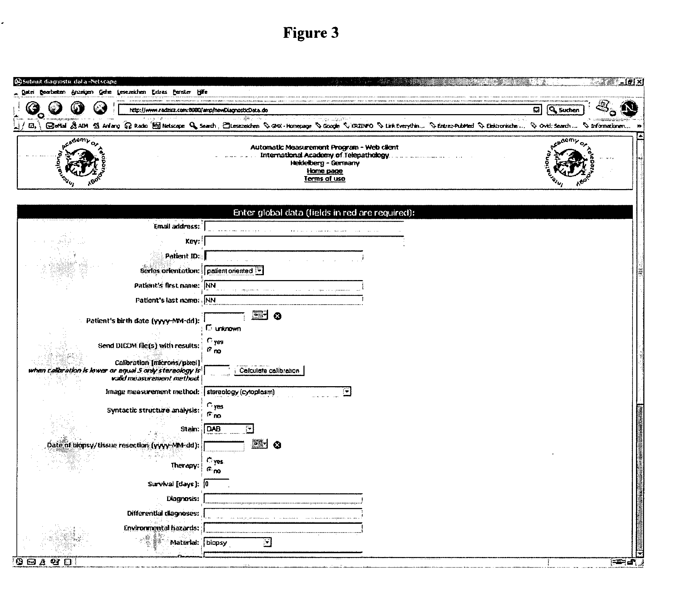

The internet platform provides the client with an internet browser interface for sending and accessing data. It includes a form to be filled in with data necessary for measurement and report. The interface checks filled data correctness and accuracy as well as images format (bmp and jpg). A copy of an example of its present form is depicted in figure 3. The data is sent as an email and collected in an internal system email queue for further processing. The proposed solution does not require the computational station to be on-line all the time. It also improves scalability and load balancing of the system as many computational stations may be used for data processing. Moreover, since the computational system sometimes requires MS Windows system with specific software installed, it reduces the risk to have this system not-exposed directly as a part of Internet portal.

### Email processing program

The email processing program receives email messages with data, extracts data and converts images to an appropriate format for measurements (.bmp), transfer the files to the chosen measurement input paths (disk queue), and control the output paths for re-submitting the results to the client. In addition, they administrate the image data bank and serve for security tasks in the internet communication (user authorisation).

In addition, it acts for control of correct email messages, checks the correct information content and provides the data security. It transfers the input data (images to be measured) into a system-internal standard and makes the images and data available for access by the measurement program.

The measurement results are re-transferred to the e-mail processing program, which then feeds the data into an image databank, provides the client-oriented form of the results, and submits the results to the client via email.

### Measurement program structure

The measurement program starts with the configuration of its necessary file data. These include the disks and paths of input and output files, the format of the files to be searched for, and the configuration of the data set up (clients data and translation of DICOM format). Having configured these internal standards, a loop is started which checks the input disk and path for incoming data and image files. The data file provides the program with the necessary measurement information, i.e. ID number, separation symbol for antibodies (#) and image numbers (_), measurement procedure, and image calibration.

The program then leaves the loop for file checking, performs the wanted measurement, and creates an output file with the measurement data. This file will be picked up by the platform program, translated into DICOM standard, and sent to the client via internet (email attachment). Images, input, output and control data are then included into an image data bank.

During the whole measurement procedure a control file is created which is erased after finishing the measurement. Having performed the measurements, a pertinent control message is implemented at the control path, which serves for documentation and which is included into the internal image data bank together with the measurement request, result and image files.

The internal loop is started again after determination of the measurement. The checking period of the loop is automatically adjusted to the time needed for measurement, i.e., if the measurement time lasts long, the checking time is prolonged. An input file checking time of 10 minutes is standard.

The measurement program components are of importance for the system and comprise the following program sets:
1. Internal check for data (images) to be measured
2 setup of internal standards
3. setup of internal control during measurement
4. check of image calibration
5. analysis of the used staining procedure (DAB, ACE, fluorescence, etc.)
6. perform measurements of
   a. stereology (chip analysis)
   b. nuclear stains (proliferation, hormone receptors, etc.)
   c. membrane stains (Herceptin, receptors, etc.)
   d. vascular stains
   e. syntactic structure analysis (b - d)
8. present the results in the client's format on an internal result file.

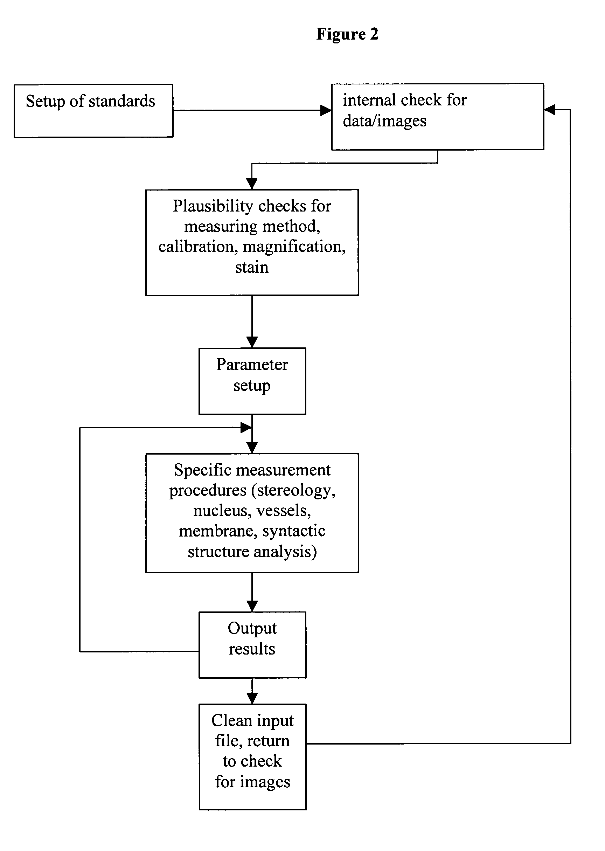

The body of the system is schematically displayed in figure 2: In detail:
The module <**setup of standards**> is an external program, which interacts with the measuring program. It is used for internal definition of file structure and disks/paths for the following purposes where:
the incoming images have to be expected,
the program controls/protocols, and the program status will be shown
the results will be transferred.
The module **<internal check for images>** periodically searches for images and data transferred by the email processing program. The periods of checks are predefined by the module <setup of standards>
The module **<plausibility check>** controls the assignment of images/stains/ with the "clinical" data (patient's ID, experiment number, etc.), the correct adjustment of image calibration with the measurement procedure, and the possibility of object segmentation.
The module **<parameter setup>** selects the **<specific measurement procedure>** and provides the **<specific measurement procedure>** with prerequisite parameters such as fluorescence, stains, magnification/calibration, number of images to be measured, etc.
The module **<internal control>** reports about the status of the measurement program.
The module **<specific measurement procedures>** is composed of five different subunits, which perform the measurements **<cytoplasm/stereology>, <nucleus>, <membrane>, <vessels>,** and **<syntactic structure analysis>.** It contains a self-adjusting segmentation procedure, which is implemented using the theory of active sampling. At present, the specific measurement procedures for immuno- and ligand-histochemistry are implemented as follows:
1. stereological features at low magnification (1.5x - 10x)
2. vascular stains (low - moderate magnification (10x - 20x)
3. nuclear stains (moderate - high magnification (20x - 40x)
4. membrane stains (high magnification (40x - 60x)

According to the diagnostic and therapeutic requirements, each measurement procedure results in a specific data output as follows:
1. stereology (volume (area) and length fractions
2. vascular (volume fractions, absolute measures, diffusion simulation, relations between cells and distance to the nearest vessel)
3. nuclear (fractions between positive/negative events, volume standardised counts, volume fractions, scores)
4. membrane stains (fractions positive/total membrane length, scores, spatial arrangement (structural entropy))

Syntactic structure analysis can be applied to the measurements procedures 2 - 4 to obtain results on clusters of cells/vessels with different spatial, size and intensity arrangements. These data permit an insight into the distribution of cells with the investigated molecule expression.

The module <**output results**> transfers the result data according to the request of the client into an internal standard, and make the data available for the <email processing program>.

The module <**clean input file**> releases the <**internal control**>**,** erases the measured files, and resets the whole <**measuring program**> into its original state.

### Internal mandatory formats:

Data sets and images are identified by their names, i.e.,
image ID and data file ID have to be identical (for example: 1234.txt, 1234.jpeg)
Image file names can be <data ID#antibody_image number>
(examples: 1234.txt; 1234#mib_1.jpeg; 1234#mib_2.jpeg; 1234#mib_3.jpeg;)
(3456.txt;3456#mib.jpeg;3456#vim.jpeg;3456#herceptin.jpeg;)
(5589.txt;5589_1.bmp;5589_2.bmp;)
(ab123.txt;ab123.jpeg)
Antibody separator: < # >;
Image number separator: <_>;
The ID number entered in the front page should be identical with the file ID;
The antibody given at the image ID is taken for the antibody named in the result file.
The separators are not permitted in the data file ID (for example: 123new.txt is correct; 123#new.txt is not permitted).

### Basic measurement procedures

The measurements are based upon a self-controlling automated program written in a visual-basic like language (DIAS51). The program searches certain disks and paths for appropriate data sets and images to be measured. Once the images and the associated data-file have been detected it starts its measurement procedure(s) according to the clients commands. The results will be presented in a <*.txt> file on a chosen result path (and disk). The disks and paths can be arbitrarily chosen by use of an included "set-up" program called parameter_set.amb.

The systems has been designed to serve for
1. individual clinical (patient oriented), and
2. serial/experimental purposes.

The *individual clinical measurement* is performed, if the user submits images of an individual patient, for example images taken from slides stained with different antibodies. The measurement result will then be patient-oriented; i.e., an individual textfile is created for each patient. This textfile can be read by general available programs such as excel, etc.. It might contain the results of different applied antibodies.

The *experimental measurement* is appropriate if an experimental series of the same antibody has to be analyzed performed on multiple different individuals. The measurement result is consecutively a single textfile which contains all individuals (lines) with an identical arrangement of the measurement data (rows).

The results of "common" measurements (procedure 1 - 4) are presented in a textfile which does not include those of potential additional structure analysis. The latter ones are given in a separate textfile which includes the surfax <syntact>.

During the measurement itself a control file is created which contains all significant information about the ongoing measurement. Once the measurement has been determinated, the control file will be erased.

Once the input and output disks and paths of the measurement program have been chosen they remain fixed for the chosen measurement time. However, they can be changed after termination the running measurement program and a consecutively restart.

Basically, the program permits measurements:
a) of multiple images assigned to one antibody and one patient
b) multiple images assigned to several antibodies and one patient
c) multiple images and one antibody assigned to different patients (experimental)
The program requires one data set file (input txt file) for each measurement procedure. The images can be the same for different measurement procedures.

Application for and use of the system
The system can be applied via the internet by a specific server address. The system requires an identification of the user (pathologist, researcher, clinician) by his name, institution, and a personal security key. Only registered individuals/institutions have access to the system. After successful registration, the client can act as follows:
a) setup of preferences
b) submit data for measurement

### Access for registration

User is identified basing on his/her email address and personal security key. User administration is now performed manually but final solution will be based on the web form. New user will have to fill the form and after his/her personal data verification personal security code will be given and the user will have an access to the system.

### Reading the image databank

The system is equipped with image data bank. This bank will be available online in Internet for registered user. Special access policy rules are created to meet all security and privacy requirements. Only the user who submit the case will have an access to it as long as he explicitly allow an access for the selected group of or all users.

### Result accessibility

Data processing results are send to the submitter by an email (with all security standards met). He is also notify about data rejection (and reason) and processing failures. The result form includes thumbnails of all submitted images, data and of course calculated measurements is send as Acrobat Reader (pdf) document. Example on is attached in appendix.

### Filling in the front page

The front page is divided into the subsets <submit new diagnostic data> and <configure preferences>.

Choosing the button <submit new diagnostic data> opens a form, which includes all switches and data needed for an appropriate measurement. Mandatory data are marked in red. They include e-mail address, key, patient's iID image measurement methods, stain, calibration (or adequate magnification data), diagnosis, material, organ. Images can be easily attached, and are not limited in number or format.

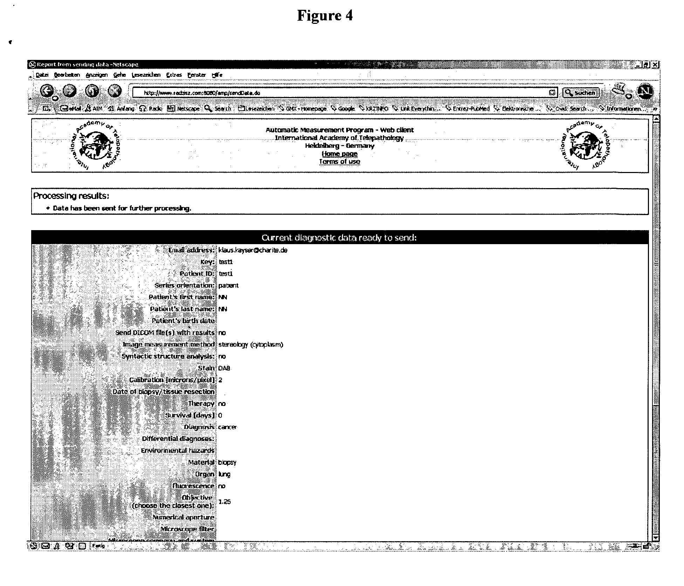

Choosing the button <configure preferences> the client can fix his default values.
The data with the attached images are automatically transferred via email to the server. Once the data have been successfully transferred to the server, the email processing program receives a corresponding message, that the data are in preparation for measurement. An example of the presented screen is shown in Figure 4.

## Claims

1. A method for the automated analysis of biological images, said method comprising the steps of
a) collecting and providing biological image data to first output means
b) transmitting said image data from said first output means to input processing means
c) processing said image data by said input processing means
d) transmitting said processed image data to automated image analysis means
e) further processing said processed image data by said automated image analysis means to generate image analysis result data
f) transmitting said image analysis result data to output processing means, and
g) further transmitting said image analysis result data to second output means.

2. Method according to claim 1, **characterised in that** the biological image data is derived from immunohistochemical images, in situ hybridisation images, immunofluorescence images, radiological images or endoscopical images.

3. Method according to claim 1 or 2, **characterised in that** the first output means comprises an interface for accessing and sending data.

4. Method according to claim 3, **characterised in that** the interface for accessing and sending data comprises an internet browser.

5. Method according to any of claims 1 to 4, **characterised in that** said transmitting of said image data to said input processing means comprises filling out a form with data necessary for measurement and report.

6. Method according to any of claims 1 to 5, **characterised in that** said image data is collected and further processed in an internal system email queue of said first output means.

7. Method according to any of claims 1 to 6, **characterised in that** said first output means are constantly on-line or not constantly on-line.

8. Method according to any of claims 1 to 7, **characterised in that** said input processing means performs steps selected from the group consisting of receiving messages with data, extracting data, converting image data into an appropriate format for further measurements, checking filled data correctness and accuracy as well as images format, transferring the data to the measurement input paths, administrating the image analysis means and serving for security tasks in the communication.

9. Method according to claim 8, **characterised in that** said input processing means comprises an email processing program.

10. Method according to any of claims 1 to 9, **characterised in that** said automated image analysis means performs steps comprising configuring the necessary file data, starting a loop checking the input disk and path for incoming data and image files, leaving the loop for file checking, performing the requested measurement, and creating an output file with the image analysis result data.

11. Method according to claim 10, **characterised in that** configuring the necessary file data comprises checking the disks and paths of input and output files, the format of the files to be searched for, and the configuration of the data set up.

12. Method according to any of claims 1 to 9, **characterised in that** the output processing means comprises the steps of picking up the file from said automated image analysis means, and translating said file into a suitable standard.

13. Method according to claim 12, **characterised in that** the suitable standard is DICOM.

14. Method according to any of claims 1 to 13, **characterised in that** said transmitting said image analysis result data to the client takes place via the internet and/or as an email attachment.

15. Method according to any of claims 1 to 13, **characterised in that** said second output means comprises an image databank, an interface or a monitor.

16. Method according to claim 15, **characterised in that** the interface comprises an internet browser.

17. Method according to any of claims 1 to 16, **characterised in that** said method is performed substantially without human intervention.

18. Method according to any of claims 1 to 17, further comprising
a) an automated internal and/or external control of image measuring systems by internally controlling of features of segmented areas according to the aim of the measurement, wherein objects with features are detected that do not match the features of the expected objects (or set of objects), and
b) providing the client with a respective output information.

19. Method according to any of claims 1 to 18, further comprising controlling of internal "program failure" by an appropriate program reaction, for example, a stop of the measurement, if an internal error occurs and a restart of the program.

20. Method according to claim 19, wherein the restart of the program comprises either a new measurement or "internal repair".

21. A data processing system for automatically analysing biological images, wherein said system comprises means for performing the method according to an of claims 1 to 20.

22. Computer program product comprising program code means stored on a tangible medium readable by a computer, the code means to execute the method according to one of the claims 1 to 20 when executed in the computer.

23. Computer readable storage medium comprising a program according to claim 22 in the form of a computer readable program code.
